# EUROPEAN PATENT APPLICATION

(11) **EP 2 468 746 A1**
(43) Date of publication of application: **27.06.2012**
(21) Application number: 10196774.3
(22) Date of filing: 23.12.2010
(51) Int. Cl.: C07D 417/04, A61K 31/5415, A61P 31/06

(54) **Benzothiazinone compounds and their use as anti-tuberculosis agents**

(71) Applicant: The University of Queensland, St.Lucia, Queensland 4072 (AU)
(72) Inventor: Cooper, Matthew, Chapel Hill, Queensland 4069 (AU); Zuegg, Johannes, Wynnum, Queensland 4178 (AU); Becker, Bernd, New Farm, Queensland (AU); Karoli, Tomislav, Kenmore, Queensland 4069 (AU)
(74) Representative: HOFFMANN EITLE

(57) **Abstract**

Novel benzothiazinone derivatives of formula (I) or a pharmaceutically acceptable salt or solvate thereof have been found to be effective against Mycobacterium tuberculosis strains and may thus be useful in the treatment of tuberculosis: 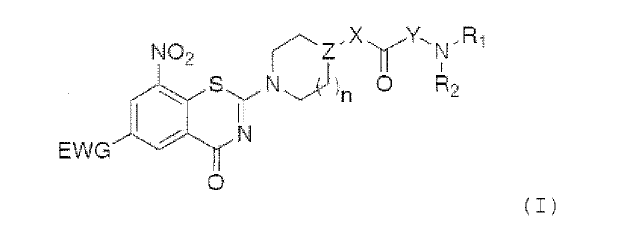 wherein
EWG (electron withdrawing group) = NO₂, CN, CF₃, F, Cl, Br, OCF₃, OH, OR, OCHF₂, COOR, wherein R is hydrogen, or a straight or branched C₁-C₄ alkyl group,
X a bond, or a straight or branched C₁-C₄ alkylene group;
Y = a bond, or a straight or branched C₁-C₄ alkylene group; wherein either one of X or Y is a bond and the other is a C₁-C₄- alkylene group,
Z = N or C,
n = 1 or 2;
R¹ = hydrogen, a straight or branched C₁-C₆ alkyl group, or a C₃-C₆ cycloalkyl group, which may be substituted with a group selected from F, Cl, Br, I or a C₁-C₄ alkoxy ;
R² = a phenyl group, a naphthyl group or a thienyl group, each of which may be unsubstituted or substituted with one or more substituent(s) which may be the same or different from each other, selected from the group consisting of F, Cl, Br, I, CN, NO₂, or a straight or branched C₁-C₆ alkyl or phenyl group, which may be substituted with a group selected from F, Cl, Br, I, or C₁-C₄ alkoxy.

## Description

### Field of the Invention

The present invention relates to novel benzothiazinones of formula (I) that kill *Mycobacterium tuberculosis* by blocking the arabinan synthesis and to a process for the preparation of the compounds of formula (I).

### Background of the Invention

The loss of human lives to tuberculosis (TB) continues essentially unabated as a result of poverty, synergy with the HIV/AIDS pandemic and the emergence of multidrug-and extensively-drug resistant strains of *Mycobacterium tuberculosis.* It is therefore necessary to continually provide innovations in this field to combat these highly dangerous infections, particularly when they are caused by strains that are resistant to the existing anti TB drugs.

WO 2007/134625 and EP Application 07013899 describe antimicrobial compounds of the benzothiazinone class and their use as antibacterial agents in infectious diseases of mammals (humans and animals) caused by bacteria, especially *Mycobacteria.*

EP 2 176 248 B1 (WO 2009/010163) describes the use of certain piperazine-substituted benzothiazinones as effective agents for the treatment and prevention of tuberculosis and leprosy caused by *Mycobacteria.*

The object of the present invention is to provide compounds that have at least the same antimicrobial efficacy as the compounds of EP 2 176 248 B1 and WO 2007/134625, and in addition a superior profile of pharmacologically important properties such as stability, solubility and toxicity. To this end the present invention provides compounds of formula (I) which differ from the prior art compounds in their particular selection of substituents.

### Summary of the invention

The present invention provides benzothiazinone compounds of formula (I), which are biologically effective against mycobacteria strains that are known to cause tuberculosis and leprosy. In addition the compounds of the present invention have a very good and generally improved profile of pharmacologically relevant properties such as stability, solubility and toxicity over known compounds of the same class. The present compounds are suitable for the prevention and treatment of tuberculosis and leprosy caused by the multidrug- and extensively-drug resistant strains of *Mycobacterium tuberculosis.*

### Disclosure of the invention

In a first embodiment, the compounds of the present invention are compounds of formula (I) or pharmacologically acceptable salts thereof as shown in the following 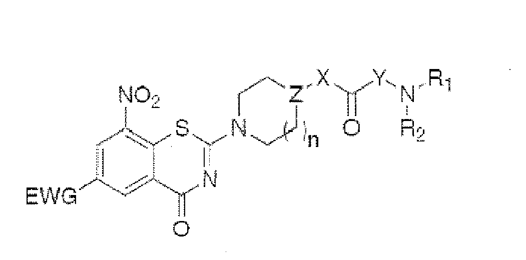 wherein

EWG (electron withdrawing group) = NO₂, CN, CF₃, F, Cl, Br, OCF₃, OH, OR, OCHF₂, COOR, wherein R is hydrogen, or a straight or branched C₁-C₄ alkyl group,
X = a bond, or a straight or branched C₁-C₄ alkylene group;
Y = a bond, or a straight or branched C₁-C₄ alkylene group; wherein either one of X or Y is a bond and the other is a C₁-C₄- alkylene group,
Z = N or C,
n = 1 or 2;
R¹ = hydrogen, a straight or branched C₁-C₆ alkyl group, or a C₃-C₆ cycloalkyl group, which may be substituted with a group selected from F, Cl, Br, I or a C₁-C₄ alkoxy ;
R² = a phenyl group, a naphthyl group or a thienyl group, each of which is unsubstituted or substituted with one or more substituent(s) which may be the same or different from each other and are selected from the group consisting of F, Cl, Br, I, CN, NO₂, or a straight or branched unsubstituted or substituted C₁-C₆ alkyl or phenyl group, wherein the optional substituents of such alkyl or phenyl groups are selected from F, Cl, Br, I, or C₁-C₄ alkoxy.

In a second embodiment, the present invention is directed to a compound of formula (I), wherein
EWG = CF₃;
X = a bond, or a straight or branched C₁-C₄ alkylene group;
Y = a bond, or a straight or branched C₁-C₄ alkylene group; wherein either one of X or Y is a bond and the other is a C₁-C₄- alkylene group;
Z = N;
n = 1:
R¹ = hydrogen, a straight or branched C₁-C₄ alkyl group, or a C₃-C₆ cycloalkyl group;
R² = a phenyl group, which is unsubstituted or substituted with at least one substituent which may be the same or different from each other and is selected from the group consisting of F, Cl, Br, I, or a straight or branched C₁-C₄ alkyl group.

Further preferred compounds of the invention include those according to any of the first or second embodiments (the same applies for the following preferred compounds), wherein X is a methylene (-CH₂-) group or a -CHCH₃-group and wherein Y is a bond.

Further preferred compounds of the invention include those, wherein R¹ is a methyl group or a cyclopropyl group and wherein R² is a phenyl group, which is unsubstituted or substituted in para-position with a CH₃ group, F or Cl.

Further preferred compounds of the invention include those, wherein X is a -CH₂-group or a -CHCH₃-group, Y is a bond, and R¹ is a CH₃-group or a cyclopropyl group.

Further preferred compounds of the invention include those, wherein Y is a bond, X is a -CH₂ -group or a -CHCH₃-group and R² is a phenyl group, which is unsubstituted or substituted in para-position with a CH₃ group, F or Cl.

Yet further preferred compounds of the invention include those wherein R¹ is a CH₃-group, or a cyclopropyl group, R² is a phenyl group, which is unsubstituted or substituted in para-position with a CH₃ group, F or Cl, X is a -CH₂-group, or a -CHCH₃-group and Y is a bond.

The most preferred compounds according to formula (I) are selected from the group consisting of the following compounds and their pharmaceutically acceptable salts: 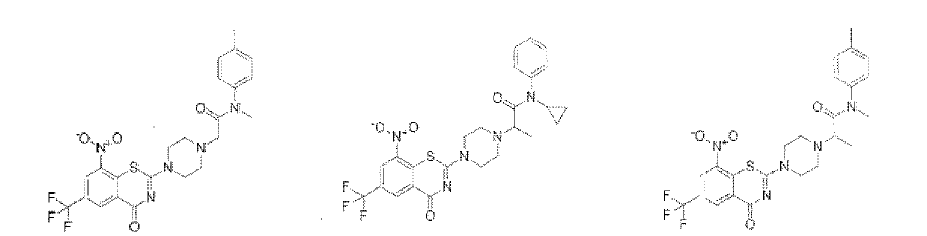 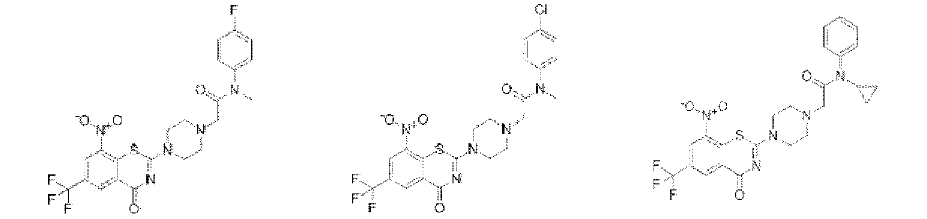 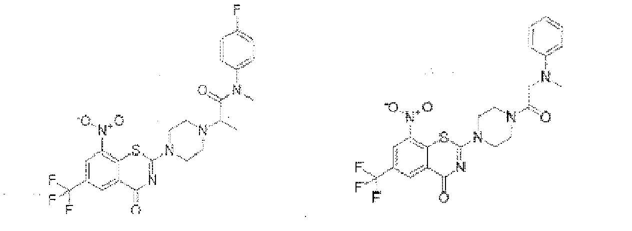 and

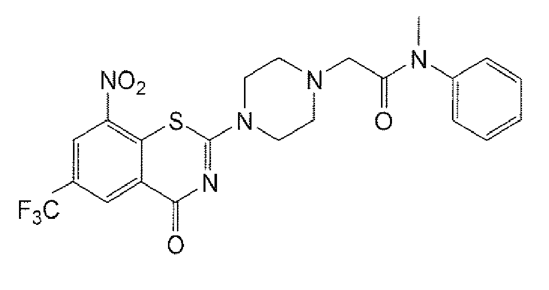

The last compound, hereinafter designated as Compound A, is presently particularly preferred.

Pharmaceutically acceptable salts of either of the above compounds are likewise preferred. They may help to improve the solubility and bioavailability of the free compounds described above.

Compounds of the present invention were found to be effective as antimicrobial agents, particularly against various strains of *mycobacteria* and thus are potential candidates for inclusion in single or combination therapies for both drug-sensitive and extensively-drug resistant TB. Compounds of formula (I) or their pharmaceutically acceptable salts are therefore useful in a method for preparing a pharmaceutical composition, particularly a pharmaceutical composition for use in a method for treating or preventing a microbial infection in mammals, particularly a tuberculosis or leprosy infection.

The present invention therefore also provides a pharmaceutical composition, particularly a tuberculosis medicament, containing a compound of formula (I) or a pharmaceutically acceptable salt thereof as shown above as the active ingredient, optionally together with one or more pharmaceutically acceptable excipients. In addition, such a composition may contain other antimicrobial compounds for which an activity against mycobacteria has been demonstrated.

The invention is further directed to a process for the preparation of compounds of formula (I) comprising the reaction of an S-methyl benzothiazinone derivative of formula (II) with a suitable amine of formula (III) (wherein all variable residues are as defined in formula (I)) to form a compound of formula (I). Such a process is shown in Scheme 1 below:

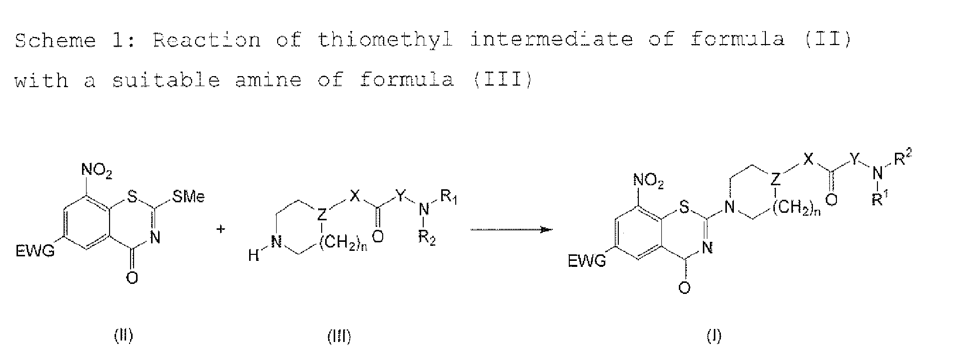

In this reaction, a nucleophilic addition of an amine of formula (III) to the thiomethyl benzothiazinone core (II) is performed. This synthetic route allows for a very convenient point of convergence in the synthesis as the benzothiazinone core can be prepared on large scale and any number of amines can then be added. The reaction of (II) with (III) is conveniently carried out in a suitable organic solvent, e.g. a lower alcohol having 1-4 carbon atoms, most preferably ethanol, or in another polar organic solvent. The reaction is conveniently carried out under slight heating, e.g. at 40-80°C, preferably at 55-65°C.

The synthesis of (II) can be accomplished in four steps starting e.g. from 4-chlorobenzotrifluoride (= p-chloro trifluoromethyl benzene). 4-Chlorobenzotrifluoride is carboxylated by selective lithiation and trapping with carbon dioxide. Nitration, conversion to the amide via the acid chloride, thiazole ring closure using carbon disulfide and methylation yields the thiomethyl key intermediate (II). This intermediate is then reacted directly with the desired amine or a salt thereof to give the compounds of formula (I). The entire process is summarized in Scheme 2.

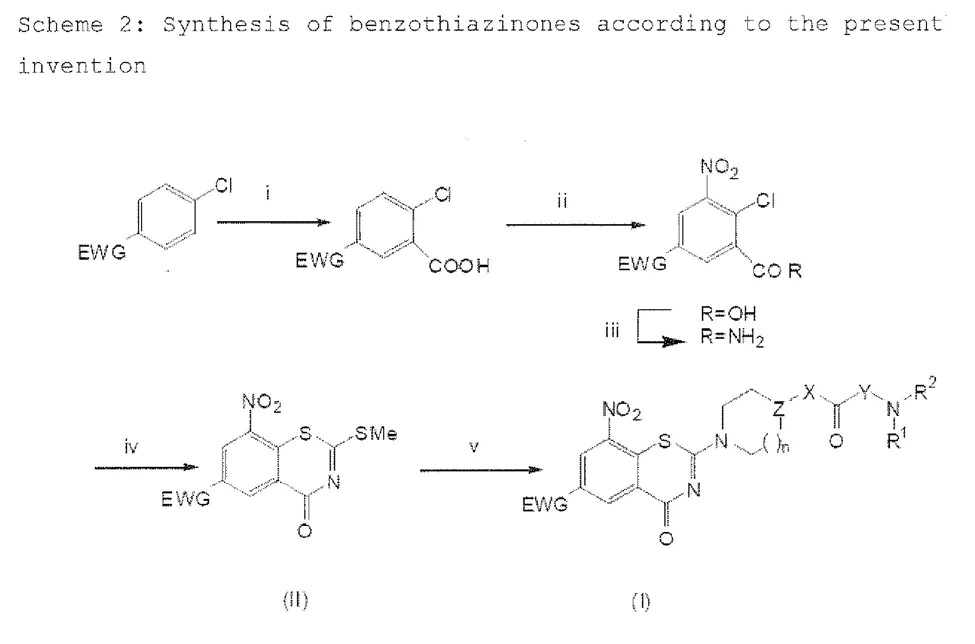

Suitable reaction conditions for each of the above steps are shown below:
i) n-BuLi, TMEDA then CO₂; ii) H₂SO₄, HNO₃; iii) SOCl₂, DMF then NH₃; iv) NaOH, CS₂, CH₃I, DMSO; v) amine of formula (III) - see below -, EtOH, 60°C.

Subsequently, the compound of formula (I) may be converted into one of its pharmaceutically acceptable salts, particularly a pharmaceutically acceptable acid addition salt thereof, by reacting the compound with a pharmaceutically acceptable acid such as hydrochloric acid, citric acid or the like.

The obtained compound may further be purified by conventional methods such as crystallization, chromatography or the like.

Further processes for synthesizing the compounds of formula (I) can be taken from the aforementioned WO 2009/010163 and WO 2007/134625 *mutatis mutandis.* Starting materials or useful intermediate can be well-known polysubstituted 2-chloro(bromo)-benzcarboxamides, many of which are described in the literature or can be easily prepared by analogous methods (Isaew S. G. Farm. Zh. (Kiev), 2000, 52; Makosza M., Nizamov S. Org. Prep. and Proced. Int., 1997, 29, 707; Nerin C., Tornes A. R., Domento C., Cacho J. J. Agr. and Food Chem., 1996, 44, 4009; Thiel W., Mayer R., Jauer E.-A., Modrow H., Dost H. J. Prakt. Chem., 1986, 328, 497; Yokoyama M., Yoshida S., Imamoto T. Synthesis, 1982, 591; Romanowski J., Eckstein Z. Pol. J. Chem., 1984, 58, 263; Nisato D., Sacilotto R., Frigerio M., Boveri S., Palmisano G., Lesma G. Org. Prep. Proced. Int., 1985, 17, 75; Oikawa N., Nakagawa Y., Nishimura K., Ueno T., Fujita T., Peptic. Sci., 1994, 41, 139; Welch D.E., Baron R.R., J. Med. Chem., 1969, 12, 299; Fuller R.W., Molloy B.B., Day W.A., Roush B.W., March M.M., J. Med. Chem., 1973, 16, 101 and many others). Likewise, the amines of formula (III) can be prepared by standard methods of piperazine or piperidine chemistry.

Pharmaceutically acceptable salts of compounds of formula (I) form a further aspect of the present invention. They particularly include acid addition salts such as the hydrochloride or hydrobromide or toluene sulfonate.

The compounds of the present invention can be used in medicaments, particularly oral or parenteral medicaments such as tablets, capsules, injection or infusion solutions. Optionally, and in some cases preferably, such medicaments also contain one or two further antibacterial agents that have activity against mycobacteria, such as TMC 207 or other diaryl quinolines, SQ 109 (= N-Adamantan-2-yl-N'-((E)-3,7-dimethyl-octa-2,6-dienyl)-ethane-1,2-diamine), bicyclic nitroimidazole compounds, INH, isoniazid, rifampicin or pyrazinamide. Such combination medicaments form a further aspect of the present invention. The compounds can be used in dosages from 0.001 - 1000 mg/kg body weight; the exact dosage will be determined by the attending physician and will also depend on the particular compound used.

A method for treating a mycobacterium infection by administering to a patient in need thereof a therapeutically effective dose of a compound of formula (I) is a further aspect of the present invention. Likewise, the present invention further envisages a compound of formula (I) for use in therapy, particularly in the treatment of infections and most particularly in the treatment of infections caused by mycobacteria such as in the treatment of tuberculosis.

### Synthesis Examples

In the following an exemplary synthesis of compounds of formula (I) is described in more detail.

Preparation of 2-chloro-5-trifluoromethylbenzoic acid 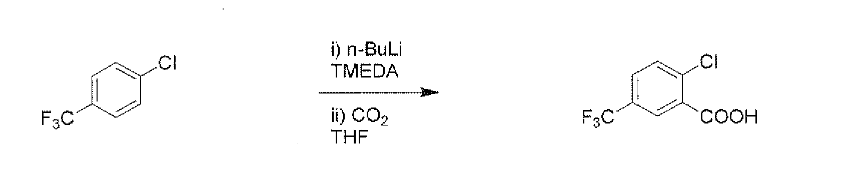 n-BuLi (2.5M in hexanes, 22 mL, 55 mmol) was added over 8 minutes to a stirred solution of 4-chlorobenzotrifluoride (9.177 g, 50.8 mmol) and TMEDA (6.3030 g, 54.2 mmol) in THF (89 mL) at -78°C under nitrogen. After 52 min the solution was transferred via cannula into dry ice (∼200g) over 20 min. Care was required to sufficiently lag the cannula as the anion solution was found to darken rapidly on slight warming. The mixture was allowed to warm to ambient temperature with stirring and was evaporated (<30°C) to give and orange/yellow solid. The solid was dissolved in water (70 mL) and washed with diethyl ether (3x30 mL). The aqueous layer was acidified to pH 1 and extracted with DCM (3x30 mL). The organic layer was evaporated to dryness (< 30°C) and the residue was dissolved in refluxing hexanes, hot filtered and crystallised by cooling to 4°C overnight. The sandy coloured solid was isolated by vacuum filtration (4.5243 g). The mother liquors were concentrated by half and a second crop of lemon yellow crystals was obtained (2.2998 g). The products were dried under vacuum to give 4.5030 g and 2.2093 g respectively, giving a combined yield of 6.712 g (59 %). LCMS: Rₜ = 5.94 min, first crop = 96.3 A% @ 254 nm, second crop 95.1 A%, [M+H]⁺ 225.0, with no starting material detected.

### Preparation of 2-chloro-3-nitro-5-trifluoromethylbenzoic acid

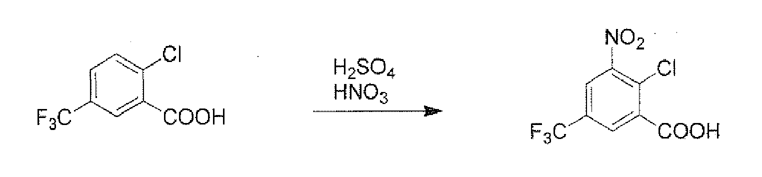

2-Chloro-5-trifluoromethylbenzoic acid (6.5044 g, 28.965 mmol) was added to a solution of sulfuric acid (23 mL) and fuming nitric acid (3.2 mL), with an overhead stirring. The mixture was warmed to 90°C over 26 min then a further 10 mL of sulfuric acid was added to the thick mixture and stirring was continued. After 45 min at 90°C the mixture was cooled to ambient temperature and poured into crushed ice (150 g), rinsing forward with icy water (50 mL). The product was isolated by vacuum filtration, washed with cold water, and dried *in vacuo* to give 7.3752 g (94.7 %). LCMS: Rₜ = 5.74 min, 100 A% @ 254 nm, [M-H]⁻ = 268.0. Co- injection of the product and starting material revealed the compounds were baseline separated.

### Preparation of 2-chloro-3-nitro-5-(trifluoromethyl)benzaroide

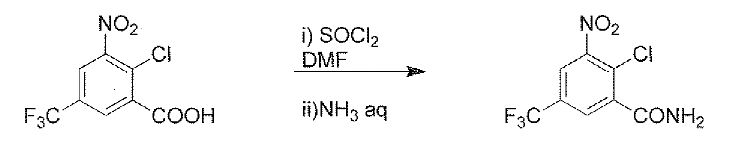

A mixture of 2-chloro-3-nitro-5-trifluoromethylbenzoic acid (1.0015g, 3.7 mmol), chloroform (5 mL), DMF (375 µL) and thionyl chloride (630 µL, 8.6 mmol) was refluxed under N₂ for 3 hr. The mixture was cooled to ambient temperature and evaporated to dryness (<30°C) . The residue was slurried in acetonitrile (2 mL) and ice cold ammonium hydroxide solution (12 mL) was added (*vigorous reaction!*). The mixture was stirred for 30 min at 0°C then the precipitate was isolated by vacuum filtration, and washed with cold water. The product was dried overnight *in vacuo* to give the desired product as a white solid 0.8206 g (82.1 %). LCMS: Rₜ = 5.60 min, 100 A% @ 254 nm, [M+H]⁺ = 269.0, [M+H]⁺ = 269.0. ¹H NMR (400 MHz, CDCl₃) δ: 8.03 (dq, 1H, *J* = 0.6, 2.2), 7.88 (dq, 1H, *J* = 0.7, 2.2), 7.84 (br s, 1H) , 7.29 (br s, 1H).

### Preparation of 2-(methylthio)-8-nitro-6-(trifluoromethyl-4H-benzo[e][1,3]thiazin-4-one (BTZ-SMe)

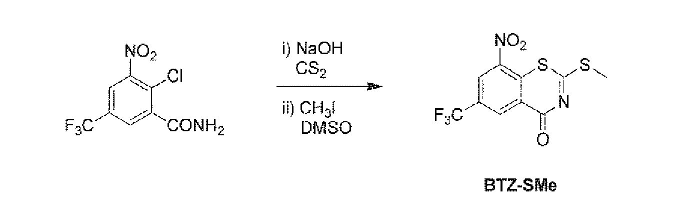

Powdered sodium hydroxide (0.234 g, 5.85 mmol) is added to a solution of 2-chloro-3-nitro-5-(trifluoromethyl)benzamide (0.800 g, 3.0 mmol) in DMSO (2.8 mL). Immediately thereafter, carbon disulfide (0.55 mL, 8.9 mmol) is slowly added to the solution at <10°C. After 15 min diiodomethane (3.0 mmol) is slowly added and formation of a solid is observed. The reaction mixture is stirred for further 35 min and then water (18 mL) is added at <20°C. The precipitated yellow solid is isolated by vacuum filtration (0.1950 g). LCMS: Rₜ = 7.03 min, 82.6A% @ 254 nm. The crude product was stirred out from ethyl acetate (2.09 g) to give BTZ-SMe as pale lemon crystals, yield = 63%. LCMS: Rₜ = 7.04 min, 99.3 A% @ 254 nm, [M+H]⁺ = 323.0. ¹H NMR (400 MHz, d₆-DMSO) δ: 8.96 (br s, 1H), 8.83 (br s, 1H), 2.76 (s, 3H).

### Reaction of the thiomethyl intermediate (II) with amine (III)

In the following starting from a compound of formula (II), a general procedure for reaction of an amine of formula (II) with a thiomethyl intermediate of formula (II) to obtain the compounds of formula (I) of the present invention is described.

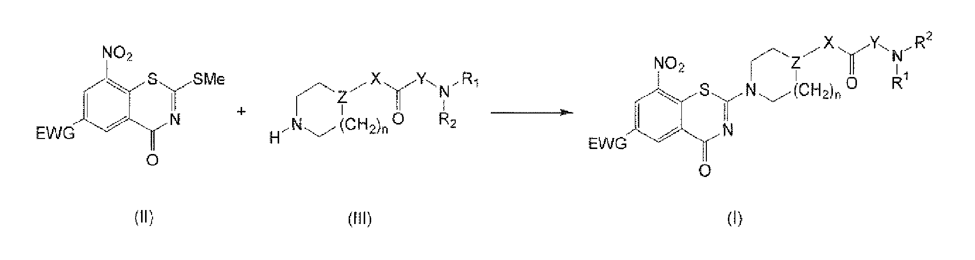

A mixture of a thiomethyl intermediate of formula (II) (1.0 eq), an amine of formula (III) (at least 1.2 eq) and ethanol (20 vol) is heated to 60°C. If the amine of formula (III) is supplied as the salt (eg as the hydrochloride salt), 1.0 - 1.5 eq of DIPEA per equivalent of acid, is included in the reaction mixture. When analysis (TLC or HPLC) shows that the reaction is complete, the reaction is cooled to ambient temperature, the solvents are removed and the product according to formula (I) is purified.

This synthetic pathway is further exemplified below using the synthesis of compound A of the present invention.

### Synthesis of Compound A

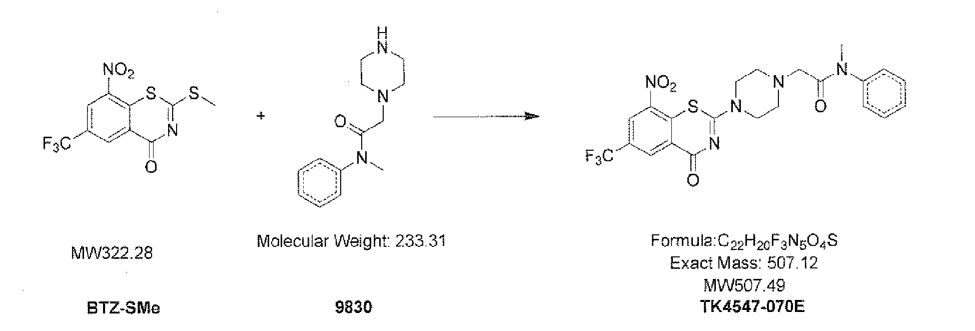

Compound A (TK4547-070E) was synthesized in line with the general procedure outlined above, by reacting the thiomethyl intermediate (BTZ-SMe) with the respective amine (9830) as shown above. The product was purified by flash chromatography over silica gel (120x10 mm, gradient elution CHCl₃ to 1:99 to 2:98 to 4:96 MeOH:CHCl₃) Rf = 0.12 @ 4:96 MeOH:CHCl₃. Isolated 15.5 mg (50 %) of off-white solid. LCMS: Rₜ = 5.57 min, 98.8 A% @ 254 nm, [M+H]⁺ = 508.0. ¹H NMR (400 MHz, CDCl₃) δ: 9.08 (dq, 1H, *J* = 0.7, 2.1), 8.75 (dq, 1H, *J*= 0.7, 2.2), 7.47-7.35 (m, 3H), 7.22-7.19 (m, 2H), 4.24-3.72 (br d, 4H), 3.29 (br s, 3H), 3.03 (br s, 2H), 2.65 (br s, 4H). HRESIMS *m*/*z* 508.1290 (calculated for C₂₂H₂₁N₅O₄F₃S, 508.1261).

### Biological Examples

Compounds of the present invention can be tested by various methods and generally show a high activity against most mycobacteria strains, i.e. a low minimum inhibitory concentration (MIC) in the nanomolar range.

### Methods

### MIC Assay Method

The protocol used is based upon that detailed by the CLSI (Clinical and Laboratory Standards Institute (CLSI) 2006. Methods for Dilution Antimicrobial Susceptibility Tests for Bacteria that Grow Aerobically; Approved Standard. 7th edn. Document M7-A6. Clinical and Laboratory Standards Institute, Wayne, PA.).

Strain: Mycobacterium smegmatis, ATCC 700084, or other mycobacterium strains (see table below)
Media: The bacteria culture and the MIC are done using two different media:
**MHB** - Mueller-Hinton Broth + 0.05% Tween80, purchased from Difco, Tween80 has been added to avoid clumping of the bacteria
**7H9** - Middlebrook 7H9 Broth, purchased from Difco, includes Tween80 by default.

### Preparation of compound solutions:

The compounds are dissolved in DSMO in a concentration of 1 mg/ml and further diluted in water to achieve the final concentration, which is used as the highest concentration for determination of MIC. This highest concentration is filled in the first row of the microtiter plate and further diluted in the culture medium for the mycobacteria (Mueller-Hinton Broth and Middlebrook 7H9 broth). All solutions are freshly prepared under sterile conditions.

Ciprofloxacin, Isoniazid and Rifampicin can be used as reference compounds for control of the assay as well as a solvent control.

Minimal inhibitory concentrations (MIC) are determined according to the CLSI guidelines using the broth microdilution method. 50 µl of the compound solution are serially diluted directly in the microtiter plate by factor two with MHB or 7H9 media. Then the wells are inocalated with 50 µl of test organisms in MHB/7H9 to give a final concentration of 5x10⁵ CFU/ml. After that the microtiter plates are incubated at 37°C for 48 h. Then 30 µl of resazurin solution is added to each well and plates are allowed to incubate at 37°C for additional 24 hrs. A change from blue to pink colour indicates reduction of resazurin and therefore bacterial growth. The MIC is defined as the lowest drug concentration that prevents this colour change.

Resazurin salt powder (Sigma) is prepared at 0.01 % (wt/vol) in distilled water, sterilized by filtration through a 0.22 µm membrane and stored at 4°C for a week.

### Cytotoxicity Assay Method

Test cells (see Table below for details) are seeded as 1x10⁴ cells per well in a clear 96 well plate in a final volume of 100 µl in media. Cells are incubated for 24 hours at 37°C, 5% CO₂ to allow cells to attach to the plates.

The compounds of the present invention are added in triplicate in a range of concentrations. For compound A e.g., the concentration started from 100µM,...and then 2-fold diluted to 0.78µM. Colistin and Tamoxifen were applied as controls.

The cells are incubated for 24 hours at 37°C, 5% CO₂. MTT (3-(4,5-Dimethylthiazol-2-yl)-2,5-diphenyltetrazolium bromide) (Sigma) (dissolved in PBS) is added to each well to a final concentration of 0.4 mg/mL. The plate is then incubated for 1 hours at 37°C, 5% CO₂. Media is removed, and crystals are resuspended in 60 µl of DMSO. Shaking the plate at room temperature for 10 minutes dissolves the formazan crystals, and the absorbance is then read at 570 nm.

Results are presented as the average percentage of control ± SD for each set of duplicate wells using the following equation: Percent Viability = (ABS_{TEST} - ABS_{5%DMSO}/ / ABS_{UNTREATED} -ABS_{5%DMSO})*100. A 1% DMSO 'vehicle' control is also included to monitor the effect of DMSO on the cells (all pure compounds are diluted in 10 % DMSO, so final assay concentration is 1%).

### Solubility Measurement

The kinetic solubilities of the compounds of the present invention can be estimated using the methods described by Alsenz et al., Hoelke et al. and Lipinski et al. (Alsenz, J.; Kansy, M. High throughput solubility measurement in drug discovery and development. Advanced Drug Delivery Reviews 2007, 59, 546-567; Hoelke, B.; Gieringer, S.; Arlt, M.; Saal, C. Comparison of Nephelometric, UV-Spectroscopic, and HPLC Methods for High-Throughput Determination of Aqueous Drug Solubility in Microtiter Plates. Analytical Chemistry 2009, 81, 3165-3172; Lipinski, C. A.; Lombardo, F.; Dominy, B. W.; Feeney, P. J. Experimental and computational approaches to estimate solubility and permeability in drug discovery and development settings. Advanced Drug Delivery Reviews 1997, 23, 3-25)

In duplicate, a DMSO solution of the test compound is diluted with pH 7.4 phosphate buffer to give a final DMSO concentration of 0.5% v/v. The mixture is then shaken for 20 hr then centrifuged (3000 rpm, 30 min). The supernatant is then assayed (duplicate injection) by HPLC comparison to a three point standard curve generated by dilution of the original DMSO solution of the compound into acetonitrile at concentrations of 20, 4 and 0.8 µM. The error in the result is estimated by summing, in quadrature, the relative standard error of the slope of the standard concentration curve and the relative standard error of the injection area for the assay sample.

The results observed are at ambient temperature (20°C). Volumes were determined solely using Gilson Pipetteman Ultra pipettes.

### Bacterial strains, growth conditions and plasmids.

*M. tuberculosis* H37Rv is used as the reference strain. The recombinant strain of *M*. *tuberculosis* H37Rv expressing the green fluorescent protein (H37Rv-GFP) bears an integrative plasmid (based on Ms6) carrying a *gfp* gene constitutively expressed from the promoter *pBlaF.* Both strains are grown at 37°C in Middlebrook 7H9 broth (Difco) supplemented with 0.05% Tween 80 and albumin-dextrose-catalase (ADC) or on solid Middlebrook 7H11 medium (Difco) supplemented with oleic acid-albumin-dextrose-catalase (OADC). When required, the media are supplemented with the following concentrations of antibiotics: 20 µg/ml of kanamycin, 50 µg/ml of hygromycin. To assess BTZ efficacy on non-growing cells, the streptomycin dependent model (our unpublished work) and the nutrient starvation model are used. Drug susceptibility to clinical isolates, including MDR-and XDR-strains, is performed at the Central Institute for Tuberculosis, Moscow and at the National Reference Center for Mycobacteria, Borstel.

### BTZ efficacy in the acute animal model.

BTZ efficacy in vivo can be determined in a standard mouse infection model according to e.g. the following protocol. BALB/c mice are infected with M. tuberculosis H37Rv at a bacillary load of 5 x 10⁶ CFU by injection into the eye venous sinus, 10 BALB/c mice per group. The first day of the treatment is day 8 after the infection. Mice are dosed by gavage with 37.5, or 300 mg of BTZ, per kg body weight, in carboxymethyl cellulose formulation (0.25%), once daily, 5 times/week, for four weeks. Negative control mice died 25-28 days after infection. Compound A proved to be very effective in this model and significantly prolonged the life of the treated mice.

### Results

Further results are summarized in the following Table 1:

**Table 1:**

| Compound | Structure Properties | PhysChem Property | MIC [µg/mL] | Stability [%] | CytoTox [uM] |
|---|---|---|---|---|---|
| | MW logS | Solubility | M smeg 700084 ^{IMB} | Buffer | HUVEC (GC₅₀) |
| | | | M smeg 5G987 | Plasma (H/M) | K-562 (GC₅₀) |
| | | | M vaccae 10670 | Liver Mic | HepG2 (CC₅₀) |
| | | | M fort B | | HEK293 (CC₅₀) |
| | | | M aurum SB66 | | HeLa (CC₅₀) |
| | | | M tb MDR (BACTEC) | | |
| 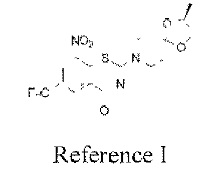 | 431 | 7 | 0.008 | 98 | 8 |
| | -4.7 | | 0.00312 | 107/97 | 11 |
| | | | 0.0004 | 98/45 | >100 |
| | | | 0.00156 | | >100 |
| | | | >100 | | >50 |
| | | | ≤0.015 | | |
| Cpd 1 of WO 2007/134625 | | | | | |
| 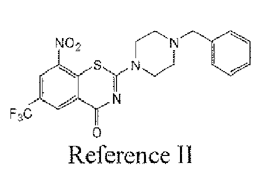 | 450 | | n.d. | | |
| | -5,9 | | 0.05 | | |
| | | | 0.05 | | |
| | | | 0.05 | | |
| | | | n.d. | | |
| | | | n.d. | | |
| (Cpd 7 of WO 2009/010163) | | | | | |
| 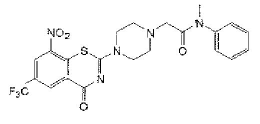 | 508 | 12 | 0.015 | 78 | 18 |
| | -5.5 | | 0.0125 | 95/115 | 30 |
| | | | 0.00312 | 13/2 | >100 |
| | | | 0.0125 | | >100 |
| | | | >100 | | 39 |
| Compound A | | | 0.03 | | |

The minimal inhibitory concentrations (MIC) of the claimed compounds against different mycobacteria are very low, and generally in the range of 1-20 ng/ml for most mycobacteria strains tested. As shown in the above table, the MIC for one of the most preferred compounds of the present invention, Compound A, against MDR *M*. *tuberculosis* and *Mycobacterium smegmatis* were 30 ng/ml (59 nM) and 12 ng/ml (25 nM), respectively (Table 1), which compares favorably with those of the existing TB drugs, isoniazid, INH, (0.02 -0.2 µg/ml) and ethambutol, EMB, (1 -5 µg/ml) and is also at least equal as the activity of compound 7 of WO 2009/010163 (50 ng/ml).

The compounds of the present invention, and particularly Compound A, appear to be suitable candidates for development into a sterilizing anti tuberculosis (TB) drug. It is currently assumed that the compounds of formula (I) act on the enzyme decaprenylphosporyl-β-D-ribose 2'-epimerase which plays a role in the arabinan synthesis. However, the present invention is not bound by this theory. The suspected mode of action is supported by the fact that neither compound A nor the Reference compound I are active against M. aureum strain SB66, which is known to be resistant against agents that target this enzyme.

In addition to their excellent antibacterial activity in vitro and in experimental models, the compounds of the present invention unexpectedly also have a favourable profile of physicochemical properties, such as lower cytotoxicity, better water solubility and good plasma stability.

The compounds of the present invention can therefore be expected to be useful in the treatment of tuberculosis and other diseases caused by mycobacteria, particularly by *M. tuberculoses* and *M*. *smegmatis,* and most particularly tuberculosis caused by multidrug-resistant strains of mycobacteria.

## Claims

1. A compound of formula (I) or a pharmaceutically acceptable salt thereof, 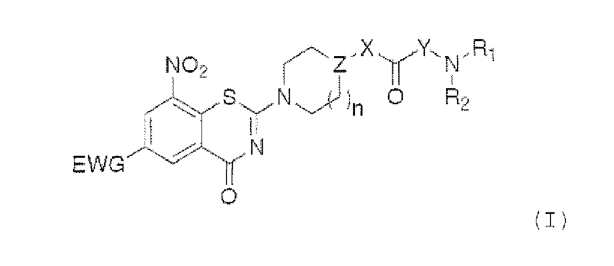 wherein
EWG (electron withdrawing group) = NO₂, CN, CF₃, F, Cl, Br, OCF₃, OH, OR, OCHF₂, COOR, wherein R is hydrogen, or a straight or branched C₁-C₄ alkyl group,
X = a bond, or a straight or branched C₁-C₄ alkylene group;
Y = a bond, or a straight or branched C₁-C₄ alkylene group;
wherein either one of X or Y is a bond and the other is a C₁-C₄-alkylene group,
Z = N or C,
n = 1 or 2;
R¹ = hydrogen, a straight or branched C₁-C₆ alkyl group, or a C₃-C₆ cycloalkyl group, each of which may be unsubstituted or substituted with a group selected from F, Cl, Br, I or a C₁-C₄ alkoxy ;
R² = a phenyl group, a naphthyl group or a thienyl group,
each of which may be unsubstituted or substituted with one or
more substituent(s) which may be the same or different from each other and are selected from the group consisting of F, Cl, Br, I, CN, NO₂, or a straight or branched C₁-C₆ alkyl or phenyl group, each of which may be substituted with a group selected from F, Cl, Br, I, or C₁-C₄ alkoxy.

2. The compound of formula (I) or a pharmaceutically acceptable salt thereof according to claim 1, wherein
EWG = CF₃;
X = a bond, or a straight or branched C₁-C₄ alkylene group; Y = a bond, or a straight or branched C₁-C₄ alkylene group; wherein either one of X or Y is a bond and the other is C₁-C₄-alkylene group;
Z = N;
n = 1;
R¹ = hydrogen, a straight or branched C₁-C₄ alkyl group, or a C₃-C₆ cycloalkyl group;
R² = a phenyl group, which is unsubstituted or substituted with at least one substituent which may be the same or different from each other and is selected from the group consisting of F, Cl, Br, I, and a straight or branched C₁-C₄ alkyl group.

3. A compound according to any of the preceding claims or a pharmaceutically acceptable salt thereof, wherein X is a -CH₂-group, or a -CHCH₃-group and wherein Y is a bond.

4. A compound according to any one of the preceding claims or a pharmaceutically acceptable salt thereof, wherein R¹ is a CH₃-group, or a cyclopropyl group and wherein R² is a phenyl group, which is unsubstituted or substituted in para-position with a CH₃-group, F or Cl.

5. A compound according to any one of the preceding claims or a pharmaceutically acceptable salt thereof, wherein X is a -CH₂ -group, or a -CHCH₃-group, Y is a bond, and R¹ is a CH₃- group or a cyclopropyl group.

6. A compound according to any one of the preceding claims or a pharmaceutically acceptable salt thereof, wherein Y is a bond, X is a -_{C}^{H}₂ -group, or a -CHCH₃-group and R² is a phenyl group, which is unsubstituted or substituted in para-position with a CH₃ group, F or Cl.

7. A compound according to any one of the preceding claims or a pharmaceutically acceptable salt thereof, wherein R¹ is a CH₃-group, or a cyclopropyl group, R² is a phenyl group, which is unsubstituted or substituted in para-position with a CH₃ group, F or Cl, X is a -CH₂ -group, or a -CHCH₃-group and Y is a bond.

8. A compound according to formula (I) or a pharmaceutically acceptable salt thereof which is selected from the group consisting of: 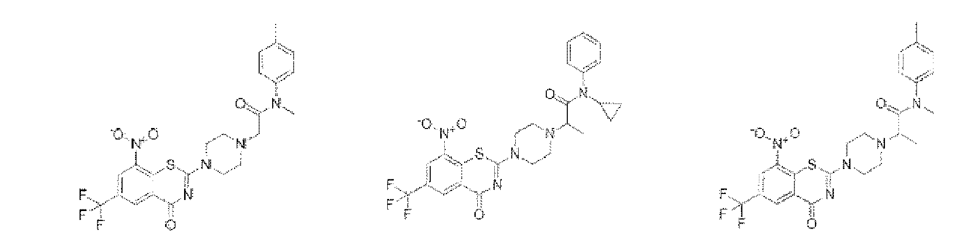 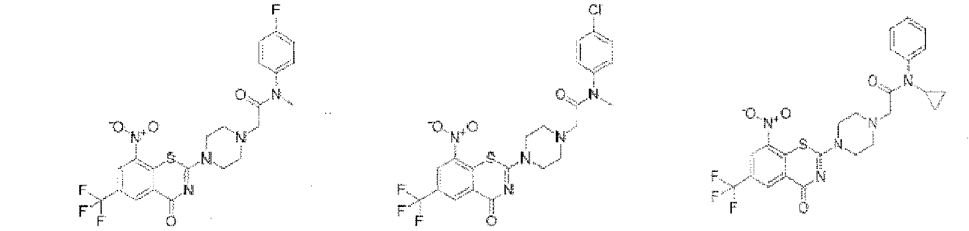 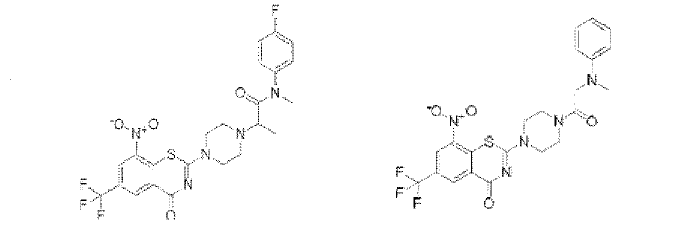 and

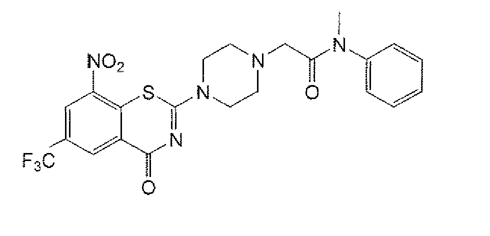

9. A compound according to claim 2 having the following formula 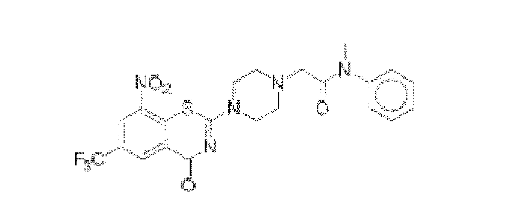 or a pharmaceutically acceptable salt thereof.

10. A compound of formula (I) or a pharmaceutically acceptable salt thereof according to any of the preceding claims for use in a method for preparing a medicament.

11. A compound of formula (I) or a pharmaceutically acceptable salt thereof according to any of the preceding claims for use in a method for treating or preventing a microbial infection in mammals.

12. The compound for use according to claim 11, wherein the microbial infection is a tuberculosis or leprosy infection.

13. A process for the preparation of a compound of formula (I) according to claim 1, comprising the steps of:
a) reacting a thiomethyl intermediate according to formula (II) 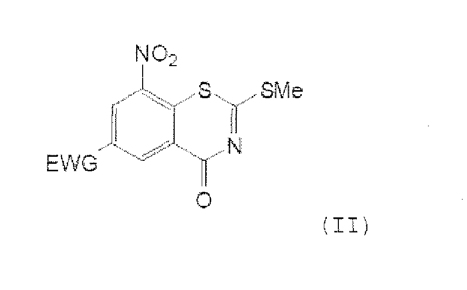 with an amine according to formula (III)or a salt thereof 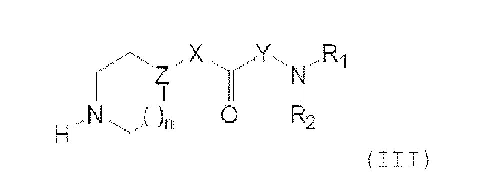 to obtain a compound of formula (I), 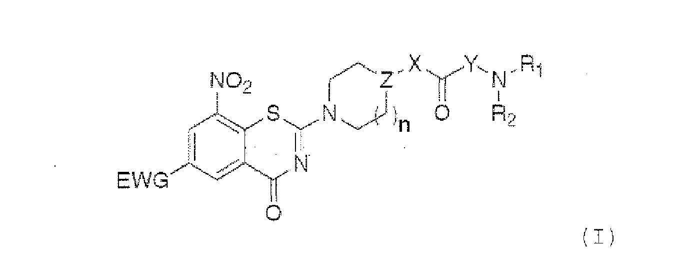 wherein
EWG (electron withdrawing group) = NO₂, CN, CF₃, F, Cl, Br, OCF₃, OH, OR, OCHF₂, COOR, wherein R is hydrogen, or a straight or branched C₁-C₄ alkyl group,
X = a bond, or a straight or branched C₁-C₄ alkylene group; Y = a bond, or a straight or branched C₁-C₄ alkylene group;
wherein either one of X or Y is a bond and the other is a C₁-C₄-alkylene group,
Z = N or C,
n = 1 or 2;
R¹ = hydrogen, a straight or branched C₁-C₆ alkyl group, or a C₃-C₆ cycloalkyl group, which may be substituted with a group selected from F, Cl, Br, I or a C₁-C₄ alkoxy ;
R² = a phenyl group, a naphthyl group or a thienyl group, each of which may be unsubstituted or substituted with one or more substituent(s) which may be the same or different from each other, selected from the group consisting of F, Cl, Br, I, CN, NO₂, or a straight or branched C₁-C₆ alkyl group, which may be substituted with a group selected from F, Cl, Br, I, or C₁-C₄ alkoxy, and
b) optionally converting the compound of formula (I) into one of its pharmaceutically acceptable salts.

14. The process for the preparation of a compound of formula (I) according to any of claims 13, wherein the reaction is performed in ethanol at 55-65°C.

15. A pharmaceutical composition containing a compound of formula (I) according to any of claims 1-10 or a pharmaceutically acceptable salt thereof.
